# EUROPEAN PATENT APPLICATION

(11) **EP 0 522 428 A1**
(43) Date of publication of application: **13.01.1993**
(21) Application number: 92111124.1
(22) Date of filing: 01.07.1992
(51) Int. Cl.: C12P 1/00, C12N 9/48, C12N 9/62, C12P 21/00

(54) **Prolyl endopeptidase and production thereof**

(30) Priority: 04.07.1991 JP 164589/91
(71) Applicant: N.V. VANDEMOORTELE INTERNATIONAL, B-8500 Kortrijk (BE)
(72) Inventor: Araki, Hideo, Kitasoma-gun, Ibaraki (JP); Ouchi, Hiroko, Kashiwa-shi, Chiba (JP); Uesugi, Shigemi, Kitasoma-gun, Ibaraki (JP); Hashimoto, Yukio, Kashiwa-shi, Chiba (JP); Shimoda, Tadahisa, Tsukuba-gun, Ibaraki (JP)

(57) **Abstract**

The present invention relates to a method to produce prolyl endopeptidase originating from Aspergillus oryzae FS1-32 (deposited with Fermentation Research Institute, Agency of Industrial Science and Technology, No. 12193). Prolyl endopeptidase is obtained by cultivation of Aspergillus oryzae FS1-32 in a culture medium containing tannic acid. The thus obtained prolyl endopeptidase has an optimum pH of 5 and an optimum temperature of 37°C, is stable at pH 4 to 7, and retains 80% of its initial activity even after heating at pH 5 and 52°C for 1 hour. Having its optimum pH in the acid region, the prolyl endopeptidase can be used under acid conditions for the inhibition of microbial propagation in its industrial application to remove the bitter taste of peptide.

## Description

### Background of the invention

The present invention relates to a new propyl endopeptidase originating from microorganisms and also to a process for producing the same.

There are several kinds of commercial proteases in use for the production of peptides. These proteases are poor in ability to cleave the bond before or after proline. Therefore, they yield peptides containing proline, which taste bitter. This is a problem involved in the production of peptides.

It has been reported that prolyl endopeptidase originates from animals as well as Basidiomycota and bacteria. However, the one originating from microorganisms poses a problem associated with putrefaction in the reaction vessel after prolonged reaction under neutral conditions, because it has the optimum pH around neutrality.

### Objects of the invention

The present invention was completed in view of the foregoing. It is an object of the present invention to provide a prolyl endopeptidase having its optimum pH in the acid region and also to provide a process for producing the same.

This and other objects and advantages of the present invention will become apparent as the description of the invention proceeds.

### Detailed description of the invention

The present inventors tested many species of microorganisms for their ability to produce prolyl endopeptidase and searched them for a strain which produces propyl endopeptidase at its optimum pH in the acid region. As the result, it was found, contrary to the common knowledge, that there exists propyl endopeptidase originating from Aspergillus oryzae, strain IFO 30113. The present inventors performed mutation on this strain so that it produces more active prolyl endopeptidase. Thus there was obtained a strain of Aspergillus oryzae FS1-32 (deposited with Fermentation Research Institute, Agency of Industrial Science and Technology, No. 12193). The present invention is based on the finding of this strain.

The present invention is embodied in a microbial prolyl endopeptidase having the following physical and chemical properties.
(a) Function: capable of cleaving by hydrolysis the bond of the carboxyl group of proline existing in peptide and protein.
(b) Substrate specificity characterized by:
   (i) a relative activity for prolylparanitroanilide which is 0 when the hydrolyzing activity for CBZ-Gly-Pro-pNA (CBZ - carbobenzoxy, pNA - p-nitroanilide) is assumed to be 100; and
   (ii)a Km value of 0.29 mM for CBZ-Gly-Pro-pNA.
(c) Optimum pH: in the neighborhood of 5
(d) Optimum temperature: 37°C
(e) pH stability: stable enough to retain more than 85% of the initial activity at pH 4-7 after heat treatment at 37°C for 2 hours.
(f) Temperature stability: stable enough to retain more than 80% of the initial activity at pH 5 after heat treatment at 52°C for 1 hour.

Aspergillus oryzae FS1-32 (deposit No. 12193) can be cultivated in the usual way employed for the cultivation of mold. In other words, the culture medium may be prepared from the following ingredients.
- Carbon source: glucose, fructose, sucrose, lactose, molasses, starch, dextrin, and glycerin (which may be used alone or in combination with one another).
- Nitrogen source: ammonium sulfate, urea, peptone, meat extract, yeast extract, corn starch, acid casein, defatted soybean, and soybean protein.
- Tannic acid to induce prolyl endopeptidase (which may be substituted for economy by black tea or green tea powder containing much tannin).
- Micro-components: sodium chloride, potassium chloride, magnesium salt, calcium salt, potassium salt, iron salt, manganese salt, vitamins, and other substances to promote the growth of the microorganism and the production of prolyl endopeptidase.

The culture medium should preferably have pH 4-7. Either liquid spinner culture or solid culture may be employed, although the former is preferable. The culture temperature should preferably be 28-35°C. The incubation period is usually 4-5 days, depending upon the temperature, pH, and kind of the culture medium used. Cultivation is suspended when the production of desired prolyl endopeptidase has reached a maximum.

After the completion of cultivation, the culture medium is filtered off to separate microbial bodies, and the filtrate is processed in the usual way for the collection of prolyl endopeptidase by several procedures in combination, such as ultrafiltration, concentration under reduced pressure, salting out, precipitation by organic solvent, dialysis, gel filtration, adsorption chromatography, ion-exchange chromatography, electro-focusing, and freeze-drying. Adequate procedures should be selected taking into account the desired physical and chemical properties of prolyl endopeptidase.

The enzymatic activity of prolyl endopeptidase is determined by measuring the hydrolysis of proline at the carboxyl group which takes place when prolyl endopeptidase acts on peptide as the substrate. The enzymatic activity mentioned in this specification was determined by the following method which employs CBZ-Gly -Pro-pNA as the substrate. The enzymatic activity to liberate 1 µM of paranitroaniline in 1 minute is 1 unit.

Method for determining the activity to decompose CBZ-Gly -Pro-pNA: A substrate is prepared from 0.25 ml of 40% dioxane solution containing 2mM of CBZ-Gly-Pro-pNA and 1 ml of 1 M citric acid-disodium phosphate buffer solution (pH 5.0). To the substrate, which has previously been heated to 37°C for 10 minutes, is added 0.1 ml of the enzyme solution, and the reaction is performed at 37°C for 2 hours. To the reaction system is added a stop solution (which is 1 M potassium chloride-hydrochloric acid buffer solution (pH 2) containing 10% Triton-X100) to suspend the reaction. A control experiment is carried out by reversing the order of adding the enzyme solution and stop solution. The adsorbance at 410 nm is measured.

The prolyl endopeptidase of the present invention has the optimum pH in the acid region and also a good thermal stability. Therefore, it can be used in the acid region where the propagation of micro-organisms is inhibited. The use of this prolyl endopeptidase reduces the bitter taste of peptide which was a problem involved in the prior art technology.

The present invention is now illustrated by the following examples.

### Example 1

A culture medium (pH 4.5) was prepared from acid casein (2.54%), roasted soybean flour (0.86%), wheat bran (1.5%), corn starch (2.0%), tannic acid (1.6%), and KH₂PO₄ (2.66%). This culture medium (100 ml) was placed in a 500-ml Sakagushi flask, followed by steam sterilization. The culture medium was inoculated with Aspergillus oryzae FS1-32 (deposit No. 12193) in 5 ml of prepropagation culture medium. Shaking culture (250 rpm) was carried out at 32°C for 4 days.

After completion of cultivation, the microbial bodies were filtered off to give a filtrate (100 ml) having an enzymatic activity of 1.98 units/ml.

### Example 2

A 30-liter jar fermenter was charged with 20 liters of the same culture medium as in Example 1. After steam sterilization at 120°C for 15 minutes, the culture medium was inoculated with Aspergillus oryzae FS1-32 (deposit No. 12193) in 1 liter of prepropagation culture medium. Cultivation was carried out at 32°C for 4-5 days. After the removal of microbial bodies, the culture medium was concentrated and freeze-dried. The thus obtained enzyme was tested for the following physical and chemical properties.

### (1) Optimum pH

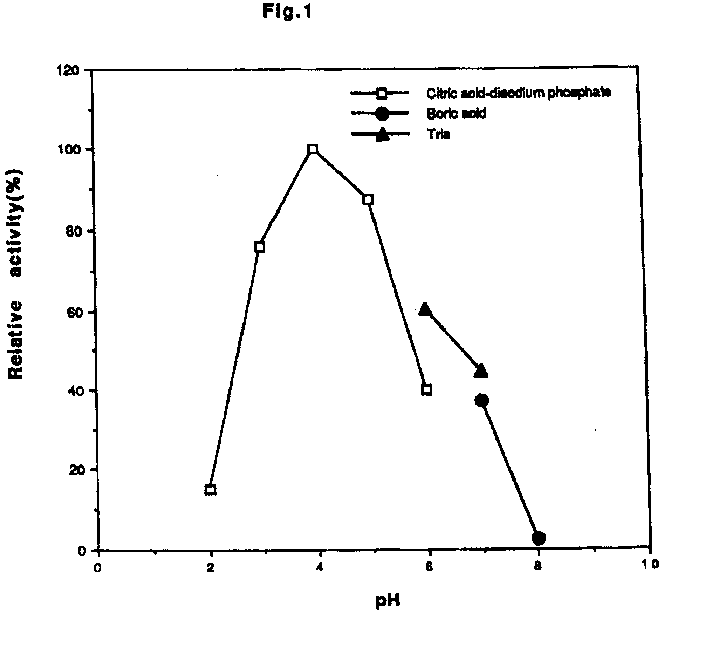

To investigate the pH dependence of activity of the enzyme sample, the above mentioned method for measuring the enzymatic activity was repeated, with the buffer solution replaced by another kind of buffer solution differing in pH. The results are shown in Fig. 1. It is noted that the optimum pH is in the neighborhood of 5.0.

### (2) Optimum temperature

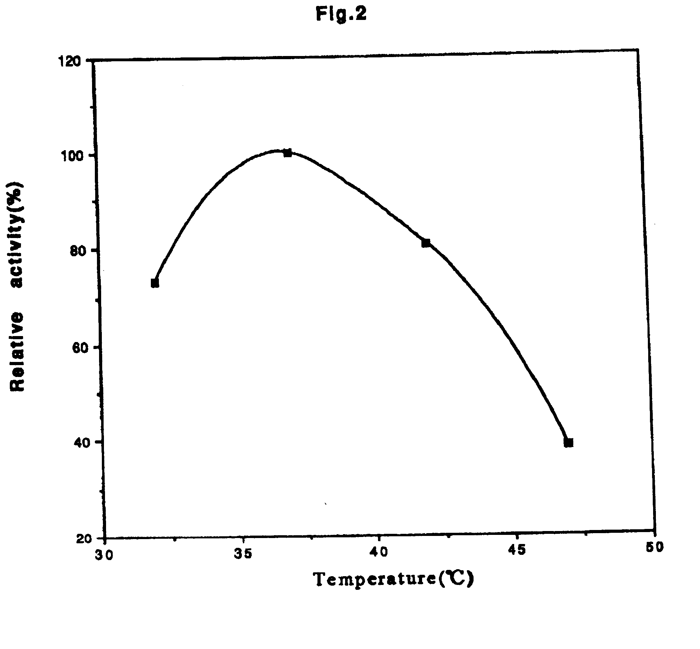

To investigate the temperature dependence of activity of the enzyme sample, the above-mentioned method for measuring the enzymatic activity was repeated at different temperatures. The results are shown in Fig. 2. It is noted that the optimum temperature is in the neighborhood of 37°C.

### (3) pH stability

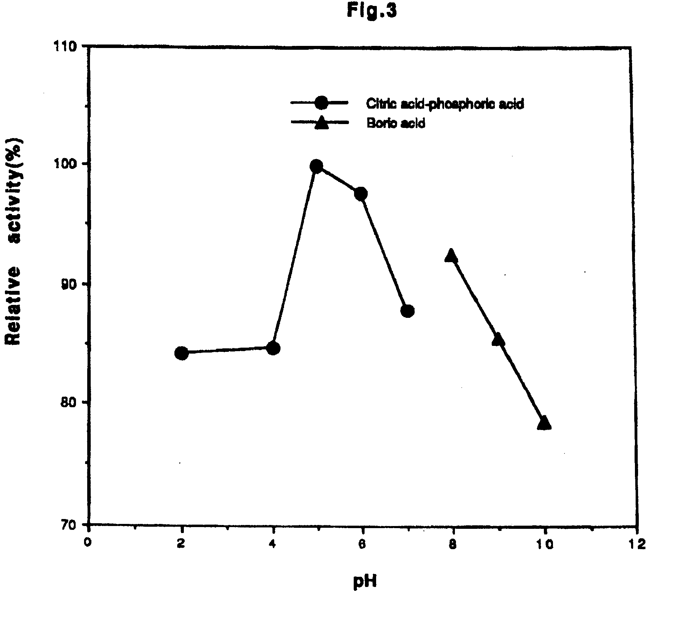

The enzyme sample was dissolved in buffer solutions differing in pH, such that the concentration of the enzyme was 1 unit/ml. After standing at 37°C for 2 hours, the enzymatic activity was measured, and the value was compared with that obtained before the test. The results are shown in Fig.3. It is noted that the prolyl endopeptidase is stable at pH 4-7.

### (4) Thermal stability

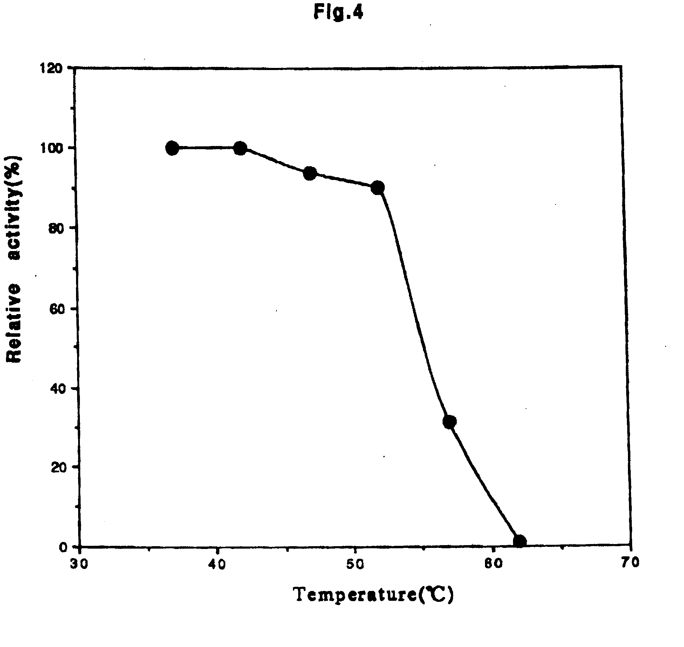

The sample was dissolved in 0.1 M citric acid-disodium phosphate buffer solution (pH 5). After standing at 30-62°C, the remaining enzymatic activity was measured. The results are shown in Fig.4. It is noted that about 80% of the activity remains even after heating at pH 5 and 52°C for 1 hour.

## Claims

1. Process for producing prolyl endopeptidase, said process comprising cultivating a species of mold belonging to the genus Aspergillus and collecting prolyl endopeptidase from the culture medium.

2. Process according to claim 1, characterized in that the mold is an Aspergillus oryzae strain.

3. Process according to claim 1 or 2, characterized in that the mold is obtained by mutation of Aspergillus oryzae strain IFO 30113.

4. Process according to any of the above claims, characterized in that the mold is Aspergillus oryzae FRI 12193.

5. Process according to any of the above claims, characterized in that prolyl endopeptidase is collected from the culture medium upon cultivation of the mold at a temperature ranging from 28 to 35°C for 4 to 5 days and removal of the microbial bodies.

6. Microbial prolyl endopeptidase capable of cleaving by hydrolysis the bond of the carboxyl group of proline existing in peptide and protein, having an optimum pH in the range 4 to 7.

7. Microbial prolyl endopeptidase according to claim 6, having the following properties:
(a) Substrate specificity characterized by:
(i) a relative activity for prolylparanitroanilide which is 0 when the hydrolyzing activity for CBZ-Gly-Pro-pNA is assumed to be 100; and
(ii) a Km value of 0.29 Mm for CBZ-Gly-Pro-pNA;
(b) Optimum pH in the range 4-7, preferably about 5;
(c) Optimum temperature of 37°C;
(d) pH stability: stable enough to retain more than 85% of the initial activity at pH 4-7 after heat treatment at 37°C for 2 hours;
(e) Temperature stability: stable enough to retain more than 80% of the initial activity at pH 5 after heat treatment at 52°C for 1 hour.
